# EUROPEAN PATENT SPECIFICATION

(11) **EP 2 379 067 B1**
(45) Date of publication and mention of the grant of the patent: **02.09.2015**
(21) Application number: 09797234.3
(22) Date of filing: 21.12.2009
(51) Int. Cl.: A61K 31/137, A61P 37/00, A61P 37/06, A61K 31/135, A61K 31/138, A61K 31/661, C07F 9/10

(54) **DOSAGE REGIMEN FOR FINGOLIMOD FOR THE TREATMENT OF MULTIPLE SCLEROSIS**
DOSIERUNGSPLAN FÜR FINGOLIMOD ZUR BEHANDLUNG VON MULTIPLER SKLEROSE
REGIME POSOLOGIQUE POUR FINGOLIMOD POUR LE TRAITEMENT DE LA SCLEROSE EN PLAQUES

(30) Priority: 22.12.2008 US 139672 P; 19.06.2009 US 218530 P; 29.09.2009 US 246715 P
(43) Date of publication of application: 26.10.2011
(60) Divisional application: 15158251.7
(73) Proprietor: Novartis AG, 4056 Basel (CH)
(72) Inventor: SCHMOUDER, Robert, East Hanover NJ 07936 (US); DUMORTIER, Thomas, CH-4002 Basel (CH); DAVID, Olivier, CH-4002 Basel (CH); LOOBY, Michael, CH-4002 Basel (CH)
(74) Representative: Rouquayrol, Céline Hélène
(86) International application number: PCT/US2009/068888
(87) International publication number: WO 2010/075239

(56) References cited:
- WO-A1-2006/058316
- WO-A2-2009/048993
- Oral fingolimod (FTY720), 0.5 or 1.25 mg, for 14 days has no effect on cardiac function: "World Congresson Treatment and Research on Multiple Sclerosis"; "Abst P507" In: SCHMOULDER R., ET AL.: "Multiple Sclerosis" 17 September 2008 (2008-09-17), Sage , London , XP009127649 vol. 14, , page S177 the whole document
- BUDDE K ET AL: "First human trial of FTY720, a novel immunomodulator, in stable renal transplant patients" JOURNAL OF THE AMERICAN SOCIETY OF NEPHROLOGY, WILLIAMS AND WILKINS, BALTIMORE, MD, US, vol. 13, no. 4, 1 January 2002 (2002-01-01), pages 1073-1083, XP003016978 ISSN: 1046-6673
- KOYRAKH LEV ET AL: "The heart rate decrease caused by acute FTY720 administration is mediated by the G protein-gated potassium channel I." March 2005 (2005-03), AMERICAN JOURNAL OF TRANSPLANTATION : OFFICIAL JOURNAL OF THE AMERICAN SOCIETY OF TRANSPLANTATION AND THE AMERICAN SOCIETY OF TRANSPLANT SURGEONS MAR 2005, VOL. 5, NR. 3, PAGE(S) 529 - 536 , XP002562406 ISSN: 1600-6135 abstract page 534, right-hand column, paragraph 1-2; figure 5
- David Kinshuck At Heartofengland Nhs Uk: "Blood pressure and diabetes", , 4 December 2006 (2006-12-04), XP055086741, Retrieved from the Internet: URL:http://medweb.bham.ac.uk/easdec/preven tion/Diabetes%20and%20Blood%20Pressure.htm [retrieved on 2013-11-05]

## Description

The present invention relates to a dosage regimen of a S1 P receptor modulator or agonist. More specifically, the present invention relates to a dosage regimen for the treatment of patients suffering from multiple sclerosis with a S1P receptor modulator or agonist.

S1P receptor modulators or agonists are compounds which signal as agonists at one or more sphingosine-1 phosphate receptors, for example, S1P1 to S1P8. The binding of an agonist to a S1 P receptor may, for example, result in the dissociation of intracellular heterotrimeric G-proteins into Gα-GTP and Gβγ-GTP, and/or the increased phosphorylation of the agonist-occupied receptor, and/or the activation of downstream signaling pathways/kinases.

S1P receptor modulators or agonists are useful therapeutic compounds for the treatment of various conditions in mammals, especially in human beings. For example, the efficacy of S1P receptor modulators or agonists in the prevention of transplant rejection has been demonstrated in rat (skin, heart, liver, small bowel), dog (kidney), and monkey (kidney) models. In addition, due to their immune-modulating potency, S1P receptor modulators or agonists are also useful for the treatment of inflammatory and autoimmune diseases. In particular, the efficacy of the S1 P receptor agonist FTY720 in the treatment of multiple sclerosis has been demonstrated in humans (as described in, for example, "FTY720 therapy exerts differential effects on T cell subsets in multiple sclerosis". Mehling M, Brinkmann V, Antel J, Bar-Or A, Goebels N, Vedrine C, Kristofic C, Kuhle J, Lindberg RL, Kappos L. Neurology. 2008 Oct 14;71(16):1261-7; and "Oral fingolimod (FTY720) for relapsing multiple sclerosis". Kappos L, Antel J, Comi G, Montalban X, O'Connor P, Polman CH, Haas T, Korn AA, Karlsson G, Radue EW; FTY720 D2201 Study Group. N Engl J Med. 2006 Sep 14;355(11):1124-40.).$ Schmouder et al.: "Multiple Sclerosis" 17 September 2008, Sage London, XP09127649 *"Oral fingolimod (FTY720), 0.5 or 1.25 for 14 days* has no *effect on cardiac function"* refers to the phase III clinical study involving daily administration of 0.5mg and 1.25mg of fingolimod in patients suffering from multiple sclerosis, and mentions the transient decrease in heart rate that occurs after initiating fingolimod therapy. This document also describes that administration of such daily doses in healthly volunteers does not affect the cardiac function.

Budde K et al J Am Soc Nephrol, 13, no 4, 1 Jan 2002; pp 1073-1083 ("First human trial of FTY720, a novel immunomodulator, in stable renal transplant patents") describes the first safety and pharmacokinetic and pharmacodynamic data obtained in human patients receiving fingolimod. These patients received a single dose from 0.25mg to 3.5mg. This document mentions that a transient, asymptomatic bradycardia occurred in some patients, and that higher doses of FTY720 were more frequently associated with bradycardia.

D. Kinshuck, at www.diabeticretinopathy.org.uk, is an educational review around blood pressure and diabetes, stressing the need of maintaining an adequate blood pressure to avoid the risk of diseases such as diabetes. This document explains that the drugs able to lower blood pressure may have strong systemic side effects and such effects can be avoided by a stepwise increase in the dose, according to the blood pressure drop needed.

Multiple sclerosis is the chief cause of neurological disability in young adults and the most common demyelinating disorder of the central nervous system. Currently available therapies, such as interferon-β and glatiramer acetate, only have modest efficacy and therefore demonstrate only marginal effects on the progression of the disease. Furthermore, these biological agents are administered parenterally and are associated with some adverse effects such as, for example, localized reactions at the injection site and pyretic symptoms. Therefore, there is a strong medical need for an effective oral treatment for multiple sclerosis.

S1 P receptor modulators or agonists may produce a negative chronotropic effect, i.e. they may reduce the cardiac rhythm, as described e.g. in "FTY720: Placebo-Controlled Study of the Effect on Cardiac Rate and Rhythm in Healthy Subjects", Robert Schmouder, Denise Serra, Yibin Wang, John M. Kovarik, John DiMarco, Thomas L. Hunt and Marie-Claude Bastien. J. Clin. Pharmacol. 2006; 46; 895. Administration of 1.25 mg of FTY720 may induce a decrease in heart rate of approximately 8 beats/min (BPM).

As a consequence of this side effect, the S1P modulator or agonist therapy may have to be initiated under close medical supervision in order to check that the cardiac rhythm is maintained at an acceptable level. This may involve the hospitalisation of patients, which makes the treatment more expensive and complicated.

Therefore, there is a need to reduce the negative chronotropic side effect that may be generated by the administration of S1 P receptor modulators or agonists, while maintaining the ability to administer an adequate dosage in order to treat or prevent the diseases for which the compound is administered. There is furthermore a need to enhance patient compliance.

### Brief Disclosure of the Invention

Surprisingly it has been found that by administering the S1P receptor modulator or agonist of the invention according to a specific dosage regimen, it is possible to reduce side effects which may be associated with the administration of such compounds. For example, administering a S1 P receptor agonist or modulator of the invention according to the specific dosage regimen of the present invention may significantly, reduce or even completely eliminate, the negative chronotropic side effect. In particular it may avoid an abrupt drop in the heart rate.

Administering a S1P receptor agonist or modulator of the invention according to the specific dosage regimen of the present invention may also significantly reduce or even completely eliminate the risks that the patients taken the S1 P receptor agonist or modulator suffer from atrio-ventricular (AV) blocks or heart pause.

Furthermore the specific dosage regimen of the present invention permits to administer a S1P receptor agonist or modulator to categories of patients for which the ratio risk/benefit may otherwise be less favourable. Such patients are for example patients susceptible to or suffering from heart failure or arrythmias, patients with high grade atrio-ventricular blocks or sick sinus syndrome, patients with a history of syncopal episodes, or patients under beta blockers or anti-arrhythmic treatment, such as patients under anti-arrhythmic drugs; or patients that have undergone an interruption or treatment holiday in the maintenance dosage regime e.g. a holiday of greater than 4 days, greater than 6, 8, 10, 12 or 14 days.

The dosage regimen of the present invention is a regimen for the initiation of therapy with the S1 P receptor modulator or agonist of the invention, which enables the standard daily therapeutic dosage range of said S1 P receptor modulator or agonist to be achieved with minimal negative chronotropic effects and/or the AV block effects possibly associated with S1 P receptor modulator therapy.

### S1P receptor modulators or agonists

The S1P receptor agonists or modulators of the invention arecompounds which, in addition to their S1 P binding properties, also have accelerating lymphocyte homing properties. For example, the compounds may elicit lymphopenia resulting from a re-distribution of lymphocytes from the circulation to the secondary lymphatic tissue, which is preferably reversible, without evoking a generalized immunosuppression. Suitably, naïve cells are sequestered and CD4 and CD8 T-cells and B-cells from the blood are stimulated to migrate into lymph nodes (LN) and Peyer's patches (PP).

A particularly preferred S1 P receptor modulator or agonist according to the invention is FTY720, i.e. 2-amino-2-[2-(4-octylphenyl) ethyl]propane-1,3-diol in free form or in a pharmaceutically acceptable salt form (referred to hereinafter as Compound A), or a prodrug thereof.

In an embodiment of the invention, the S1 P receptor modulator or agonist is FTY720 hydrochloride, as shown below:

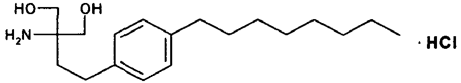

In another embodiment of the invention, the S1 P receptor modulator or agonist is FTY720-phosphate (Compound B), that is a compound of formula IIa 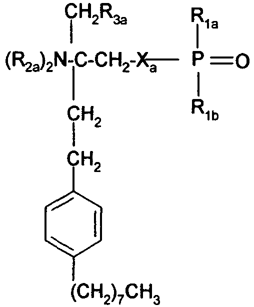 or

Wherein R₂ₐ is H, R₃ₐ is OH, Xₐ is O, R₁ₐ and R_{1b} are OH.

### Dosage Regimen

As previously stated, the present invention provides a novel dosage regimen which is adapted to minimize the negative chronotropic effects and/or the AV block effects possibly associated with S1 P receptor modulator or agonist therapy.

Heart effects include AV blocks, which include first degree AV blocks (e.g. PR intervals greater then 0.2 seconds) and second degree AV blocks e.g. first degree AV blocks. Heart effects include heart pauses e.g. heart pauses greater than 2 seconds.

According to the invention, there is provided the use of a S1 P receptor modulator or agonist of the invention in the manufacture of a medication, whereby said medication is administered in such a way that during the initial period of treatment the dosage is lower than the standard daily dosage and the dosage is increased, optionally stepwise, or only once, until the standard daily dosage dose is reached. Thereafter the treatment is preferably continued with the standard daily dosage of said S1 P receptor modulator or agonist.

Preferably during the initial period of treatment, the medication is administered in a dosage regimen such that daily decrease in heart rate (e.g. average or minimum daily heart rate) is acceptable or clinically not significant, or that the sinus rhythm of the patient is normal. For example, the daily decrease in heart rate (e.g. average or minimum daily heart rate) may be less than about 4bpm, e.g. less than about 3 bpm or less than about 2bpm.

The term "normal sinus rhythm" refers to the sinus rhythm of the patient when not undergoing treatment. The evaluation of normal sinus rhythm is within the ability of a physician. A normal sinus rhythm will generally give rise to a heart rate in the range from 60-100 bpm.

Preferably, during the initial period of treatment the dosage is lower than the standard daily dosage and the dosage is increased, optionally stepwise, or only once, until the standard daily dosage dose is reached. Thereafter the treatment is preferably continued with the standard daily dosage of said S1 P receptor modulator or agonist.

According to the invention, the "initial period of treatment" refers to the period during which the S1P receptor modulator or agonist of the invention is administered at a dosage lower than the standard daily dosage. Preferably the "initial period of treatment" starts with the first administration of said S1P receptor modulator or agonist.

The duration of the initial period of treatment may vary. In an embodiment, the initial period of treatment is up to 10 days, e.g. about a week. In a further embodiment, the initial period of treatment is 5 to 14 days, e.g. 4 to 12 days. In a further embodiment, the initial period of treatment is 7 to 10 days. In yet a further embodiment, the initial period of treatment is 4 to 7 days. In further embodiments, the initial period of treatment is selected from 10 days, or 9 days, or 8 days, or 7 days, or 6 days, or 5 days, or 4 days.

As herein above defined, standard daily dosage (also called standard daily dose) refers to the required daily maintenance dose of the drug which is given to the patients for treating or preventing the disease to be treated or prevented. Suitably, the standard daily dosage corresponds to the therapeutically effective dosage.

The therapeutically effective dosage (also called therapeutic dose) refers to the dosage of the S1 P receptor modulator or agonist of the invention which is necessary to effectively treat the intended disease or condition (i.e. so that the subject shows reduced signs or symptoms of rebound of the disease to be treated or prevented, and preferably no signs and symptoms at all).

During the initial period of treatment the S1P receptor modulator or agonist of the invention is administered at a dosage e.g. up to 4-fold less, than the standard daily maintenance dose, e.g. than the therapeutic dose.

In a preferred embodiment, there is provided the use of an S1 P receptor modulator or agonist of the invention in the manufacture of a medication, whereby said medication is administered in such a way that during the initial period of treatment (e.g. the first 10 days of treatment, e.g. the first week of treatment, or the first 5 to 14 days of treatment, the first 4 to 12 days of treatment, or the first 7 to 10 days of treatment, or the first 10, 9, 8, 7, 6 , 5 or 4 days of treatment), the dosage of said S1 P receptor modulator or agonist is given at a dosage of up to 10-fold less, e.g. up to 5-fold less, than the standard daily dose, e.g. the therapeutic dose. Optionally the dose is then raised stepwise up to the standard daily dose, e.g. the therapeutic dose, during the initial period of treatment as herein above defined.

According to the invention, medications comprise medication for patients suffering from multiple sclerosis, for example relapse remitting multiple sclerosis (RRMS) or primary progressive multiple sclerosis (PPMS), e.g. for patients suffering from RRMS.

The dosage regimen of the present invention is particularly useful for treating patents at risk of cardiac side effects, for example patients at risk of heart failure, arrythmias, patients with high grade atrio-ventricular blocks or sick sinus syndrome, patients with a history of syncopal episodes, or patients requiring or under beta blockers, or patients requiring or under anti-arrhythmic treatment, such as patients under treatment with Class Ia (e.g. quinidine, procainamide) or Class III anti-arrhythmic drugs (e.g., amiodarone, sotalol).

According to the invention, for compound A (or B), standard daily dosage is a daily dosage of about 0.5 mg.

According to the invention, the dosage of the S1 P receptor modulator or agonist of the invention during the initial period of treatment is increased stepwise in defined increments up to the standard daily dosage of said S1 P receptor modulator or agonist. Preferably, the dosage of said S1 P receptor modulator or agonist during the initial period of treatment as hereinabove defined, e.g. during the initial 10 days, e.g. 1 to 9 days, e.g. first week of treatment is increased 2-fold.

In another embodiment of the invention, the initial dosage administered during the initial period of treatment as hereinabove defined is about 4-fold less than the standard daily dosage, e.g. than the therapeutic dose, and is then increased in increments to a daily dosage which is about 2 fold less than the standard daily dosage. Thereafter, treatment is commenced at the standard daily dosage.

The same dose may be given during the first 1, 2, 3, 4, 5, 6, 7 or 8 days of treatment before the dosage is increased, preferably during the first 2 to 4 days of treatment. Suitably each subsequent incremental dosage increase is administered for 1, 2, 3, 4 or 5 days. In a particular embodiment, each subsequent incremental dosage increase is administered for 2 or 3 days.

During the initial period of treatment, i.e. before the standard daily dosage is given, the same initial dosage may be given during the first 1 to 7 days, e.g. for 2 to 5 days, e.g. the first 2 days, before the dosage is further increased, e.g. up to the standard daily dosage in one increment.

For example, the first dosage increase may occur on day 2 to day 5, e.g. day 2 to day 4, e.g. day 2, day 3, day 4 or day 5, after the first administration. The second dosage increase may occur on day 4 to 10, e.g. day 4 to 6, e.g. day 5, after the first administration. The third dosage increase may occur on day 6 to 10, e.g. day 6 or 7, after first administration.

According tothe invention, only two dosage increases occur before the standard daily dosage, e.g. the therapeutic dosage, is given.

According to the invention, the S1 P receptor modulator or agonist is Compound A or Compound B, e.g. FTY720, or a pharmaceutically acceptable salt thereof, e.g. the hydrochloride salt of FTY720.

According to the invention, the highest initial dosage of the S1 P receptor modulator or agonist of the invention, (e.g. Compound A, Compound B or a salt thereof) is 0.125mg .

According to the invention, the regimen comprises an initial daily dose of 0.125mg which is subsequently increased to a daily dose of 0.25mg during the initial period of treatment. Thereafter the treatment is continued with the standard daily dosage, e.g. 0.5mg. Again, this regimen is particularly adapted for compound A.

In a series of further specific or alternative embodiments, the present invention also provides:
1.1 The use of a S1 P receptor modulator or agonist of the invention which induces a negative chronotropic effect in heart rate, e.g. compound A, or B, or a salt or prodrug thereof, preferably compound A, a salt or prodrug thereof, in the manufacture of a medication, whereby said medication is administered in such a way to a subject that the daily decrease in heart rate (e.g. the average daily heart rate) is approximatively of 2 beats/min or less.
1.2 The use of a S1 P receptor modulator or agonist of the invention, e.g. compound A, or B, or a salt or prodrug thereof, preferably compound A, a salt or prodrug thereof, in the manufacture of a medication, whereby said medication is administered in such a way to a subject that at the day the therapeutic dosage of said S1P receptor modulator or agonist is administered the decrease in heart rate (e.g. the average daily heart rate) is approximatively of 2 beats/min or less.
1.3 The use of a S1 P receptor modulator or agonist of the invention, e.g. compound A, or B, or a salt or prodrug thereof, preferably compound A, a salt or prodrug thereof, in the manufacture of a medication, whereby said medication is administered at a lower dosage than standard dosage, e.g. 4-fold less, than the standard daily dosage, during the initial period, e.g. during the first 10 days of treatment or during the first week of treatment. The dosage is then increased stepwise up to the standard daily dosage, e.g. the therapeutic dosage, of said S1P receptor agonist.

During the initial period of treatment, e.g. the initial 10 days, e.g. 9 days, e.g. a the first week, of treatment, the daily dosage of the S1 P receptor modulator or agonist of the invention is lower than the standard dosage, and is raised stepwise two times,up to the standard daily dosage of said S1 P receptor modulator or agonist and thereafter the treatment is continued with the standard daily dosage of said S 1 P receptor modulator or agonist.
1.4 The use of a S1 P receptor modulator or agonist of the invention e.g. compound A, or B, or a salt or prodrug thereof, preferably compound A, a salt or prodrug thereof, in the manufacture of a medication, whereby said medication is administered in such a way that during the initial period of treatment, e.g. the initial 10 days of treatment, e.g. the initial first week of treatment, the dosage of said S1P receptor modulator or agonist is 4; and 2 fold less than the standard daily dosage, respectively, and thereafter the treatment is continued with the standard daily dosage of the S1P receptor modulator or agonist.
1.5. The use of a S1 P receptor modulator or agonist of the invention, e.g. compound A, B, or a salt or prodrug thereof, preferably compound A, or a salt or prodrug thereof, in the manufacture of a medication, whereby said medication is administered in such a way that during the initial 2 to 4 days of treatment the dosage of said S1 P receptor modulator or agonist of the invention is not more than ¼, of the standard daily dose of the S 1 P receptor modulator or agonist.
1.6 The use of a S1 P receptor modulator or agonist of the invention, e.g. compound A, B, or a salt or prodrug thereof, preferably compound A, or a salt or prodrug thereof, in the manufacture of a medication, whereby said medication is administered in such a way that during the initial 2 to 4 days of treatment the dosage of said S1P receptor modulator or agonist is not more than 25% of the standard daily dose of the S1 P receptor modulator or agonist.
1.7 The use of an S1 P receptor modulator or agonist of the invention, e.g. compound A, B, or a salt or prodrug thereof, in the manufacture of a medication, whereby said medication is administered in such a way that during the initial 10 days, e.g. 9 days, of treatment the dosage of said S1 P receptor modulator or agonist is lower than the standard daily dosage of said S1P receptor modulator or agonist and then the dosage is raised so that the standard daily dosage is administered two dose increases, and thereafter the treatment is continued with the standard daily dosage of said S1 P receptor agonist.
1.8 The use of a S1 P receptor modulator or agonist of the invention, e.g. compound A, or B, or a salt or prodrug thereof, preferably compound A, a salt or prodrug thereof, in the manufacture of a medication, whereby said medication is administered in such a way to a subject that the possible risk of AV block is limited or reduced to a level clinically not significant. Preferably the use is then as defined under 1.1 to 1.7.
1.9 The use of a S1 P receptor modulator or agonist of the invention, e.g. compound A, or B, or a salt or prodrug thereof, preferably compound A, a salt or prodrug thereof, in the manufacture of a medication, whereby said medication is administered in such a way to a subject that the sinus rhythm of the patient is normal. Preferably the use is then as defined under 1.1 to 1.7.
1.10 Use as defined under 1.1 to 1.9, wherein the initial period of treatment is of up to 10 days, e.g. up to 8 days , e.g. a week.
1.11 The use as defined under 1.1 to 1.10 wherein the medication is given to a patient who is at risk of heart failure.
1.12 The use as defined under 1.1 to 1.10 wherein the medication is given to a patient who is at risk of AV block.
1.13 The use as defined under 1.1 to 1.10 wherein the medication is given to a patient who experience symptoms including dizziness, fatigue, palpitations.
1.14 The use as defined under 1.1 to 1.10 wherein the medication is given to a patient with high grade atrio-ventricular blocks or sick sinus syndrome.
1.15 The use as defined under 1.1 to 1.10 wherein the medication is given to a patient with arrhythmias, e.g. requiring or under treatment with Class Ia (e.g. quinidine, procainamide) or Class III anti-arrhythmic drugs (e.g. amiodarone, sotalol).
1.16 The use as defined under 1.1 to 1.10 wherein the medication is given to a patient requiring or under beta-blocker therapy.
1.17 The use as defined under 1.1 to 1.16 wherein the medication is given to a patientsuffering from multiple sclerosis, wherein the administration of said S1P receptor modulator or agonist of the invention, e.g. compound A, or B, or a salt or prodrug thereof, preferably compound A, a salt or prodrug thereof, has been discontinuedformore than 10 days, e.g. more than 12 days, e.g. more than 14 days.
1.18 The use of FTY720, a salt or prodrug thereof in the manufacture of a medication, whereby said medication is administered, after an initial regimen as hereinabove defined, at a daily dosage of about 0,5mg.
1.19 Use as defined in 1.1 to 1.18, for treating. RRMS.
1.20 Use of FTY720 (Compound A), Compound B, or a pharmaceutically acceptable salt thereof, in the manufacture of a medicament for use in the treatment of multiple sclerosis, wherein, prior to commencing the administration of FTY720, Compound B, or a pharmaceutically acceptable salts thereof, at the standard daily dosage, the FTY720, Compound B, or a pharmaceutically acceptable salts thereof, is administered at a daily dosage which is lower than the standard daily dosage during an initial period of treatment as herein above defined (e.g. up to 10 days).

1..
   1.21 The use as defined in 1.20 wherein an initial daily dose of 0.125 mg is administered which is subsequently increased to 0.25 mg during the initial period of treatment and thereafter treatment is continued with the standard daily dosage of 0.5 mg.

In a treatment method with a S1 P receptor modulator or agonist of the invention, e.g. compound A, B or a salt or prodrug thereof, the improvement is due to said S1P receptor modulator or agonist is administered in such a way that during the initial period of treatment, e.g. the initial 10 days, e.g. 9 days, e.g. first week, of treatment, the dosage is lower than the standard dosage , e.g. 4 fold-less, e.g. 2 fold-less, than the standard daily dosage, and is increased, stepwise, up to the standard daily dosage. Thereafter the treatment is continued with the standard effective daily dosage.

Furthermore there is provided:
2.1 A method for treating a patient in need thereof such a method comprising administering a S1 P receptor modulator or agonist of the invention which induces a negative chronotropic effect in heart rate, e.g. compound A, B, or a salt or prodrug thereof, comprising administering to the subject said S1P receptor modulator or agonist in such a way that at the day the therapeutic dosage is administered the decrease in heart rate (e.g. the average daily heart rate) is clinically not significant, preferably is limited to approximatively 2 beats/min or less.
2.2 A method as defined under 2.1 comprising administering to the subject sub-therapeutic doses of the S1 P receptor modulator or agonist of the invention during the initial period of treatment.
2.3 A method for treating multiple sclerosis, e.g. RRMS, in a subject in need thereof, comprising administering to the subject, a S1 P receptor modulator or agonist of the invention, e.g. compound A, B, or a salt or prodrug thereof, at a daily dosage which is lower than the standard daily dosage and raising the daily dosage stepwise up to the standard daily dosage during the initial period of treatment, e.g. the first 10 days.
2.4 A method of ameliorating or preventing a negative chronotrophic side effect associated with a treatment using an S1 P modulator or agonist of the invention, e.g. compound A, B, or a salt or prodrug thereof, of a subject suffering from multiple sclerosis, comprising administering to the subject in need thereof, said S 1 P receptor modulator or agonist at a daily dosage which is lower than the standard daily dosage during an initial treatment period and raising the daily dosage stepwise up to the standard daily dosage.
2.5 A method as defined under 2.1 to 2.4 whereby the initial period of treatment is of up to 10 days, e.g. up to 8 days , e.g. a week.
2.6 A method as defined under 2.1 to 2.4 wherein the initial treatment period is 6-14 days e.g. 7-10 days e.g. 7 days or less, as herein above described.
2.7 The method as defined in 2.1 to 2.6, wherein the S1 P receptor modulator or agonist ois FTY720 or a pharmaceutically acceptable salt thereof, e.g. hydrochloride salt, or FTY720 phosphate, and is administered, after an initial regimen, at a daily dosage of about 0,5mg.
2.8 A method of treating multiple sclerosis, e.g. RRMS, in a patient in need of such treatment, the method comprising initiating treatment with the S1 P receptor modulator or agonist of the invention at a daily dosage which is lower than the standard daily therapeutic dosage during an initial period of treatment and thereafter commencing the administration of the S1P receptor modulator or agonist of the invention at the required standard daily therapeutic dosage.
2.9 A method of ameliorating or preventing a negative chronotrophic side effect associated with a treatment of an autoimmune disease using an S1 P modulator or agonist of the invention, e.g. compound A, B, or a salt or prodrug thereof, the method comprising administering the S1 P receptor modulator or agonist at a daily dosage which is lower than the standard daily dosage during an initial treatment period and raising the daily dosage, stepwise, up to the standard daily dosage.
2.10 A method of treating multiple sclerosis, e.g. RRMS, in a patient who is at risk of heart failure, the method comprising administering the S1 P receptor modulator or agonist of the invention, e.g. compound A, B, or a salt or prodrug thereof.
2.11 A method of treating multiple sclerosis, e.g. RRMS, in a patient who is at risk of AV block, with high grade atrio-ventricular blocks or sick sinus syndrome, the method comprising administering the S1 P receptor modulator or agonist of the invention, e.g. compound A, B, or a salt or prodrug thereof.
2.12 A method of treating multiple sclerosis, e.g. RRMS, in a patient who experiences symptoms including dizziness, fatigue, palpitations, the method comprising administering the S1 P receptor modulator or agonist of the invention, e.g. compound A, B, or a salt or prodrug thereof.
2.13 A method of treating multiple sclerosis, e.g. RRMS, in a patient with arrhythmias, e.g. requiring or under treatment with Class Ia (e.g. quinidine, procainamide) or Class III anti-arrhythmic drugs (e.g. amiodarone, sotalol); or in a patient requiring or under beta-blocker therapy, the method comprising administering the S1P receptor modulator or agonist of the invention, e.g. compound A, B, or a salt or prodrug thereof.
2.14 A method of treating multiple sclerosis, e.g. RRMS, while limiting the occurrence of symptoms including dizziness, fatigue, heart palpitations, the method comprising administering the S1 P receptor modulator or agonist of the invention, e.g. compound A, B, or a salt or prodrug thereof.
2.15 A method as defined in 2.8 or 2.14 wherein the S1 P receptor modulator or agonist is selected from Compound A (FTY720), or a pharmaceutically acceptable salt thereof, and FTY720 Phosphate (Compound B), or pharmaceutically acceptable salts thereof.

In yet another embodiment of the invention, there is provided
3.0 A S1 P receptor modulator or agonist of the invention for use in the treatment of multiple sclerosis, e.g. RRMS, wherein, prior to commencing the administration of the S1 P receptor modulator or agonist at the standard daily therapeutic dosage, said S1 P receptor modulator or agonist is administered at a daily dosage which is lower than the standard daily therapeutic dosage during an initial period of treatment.
4.0 A kit containing daily units of medication of an S1 P receptor modulator or agonist of the invention, e.g. compound A, B, or a salt or prodrug thereof, of varying daily dosage, whereby said doses are lower than the standard daily dosage.
4.1 A kit comprising units of medication of a S1 P receptor modulator or agonist as defined herein for administration according to the dosage regimen defined herein, whereby one or more low-dose units of a dose strength below the standard daily dose of the S1 P receptor agonist are provided for the initial period of treatment as herein above defined, e.g. the frst week of treatment.
4.2 A kit containing units of medication of an S1 P receptor modulator or agonist as defined herein of varying daily dosage, whereby said kit contains a) at least one of the following : about ¼, about ½, of the standard dose of the S1P receptor modulator or agonist, respectively, and b) optionally units for the standard daily dosage of the S1 P receptor modulator or agonist.

In an embodiment, the kit may comprise just one low dose unit of medication at a dosage strength corresponding to an initial dosage of the S1 P receptor modulator or agonist of the invention. A patient may then take one unit of the low dose medication for a specified number of days and then, optionally, two or more units per day on subsequent days until therapy is commenced with a unit of medication that comprises the standard daily dose of the S1 P receptor modulator or agonist of the invention.

In an alternative embodiment, the kit may comprise a number of low-dose units of medication with a range of dosage strengths so that the patient can be administered one dosage unit per day, but the amount of S1 P receptor modulator or agonist of the invention administered can be titrated upwards until therapy commences at the standard daily dosage.

The daily units of said S1 P receptor modulator or agonist may be of about 1/4 and about ½ of the standard dose of said S1P receptor modulator or agonist, respectively.

The kit may further comprise units for the standard daily dosage of the S1P receptor modulator or agonist of the invention, e.g. compound A, B, or a salt or prodrug thereof.

The kit may also contain instructions for use.

In yet another embodiment of the invention, there is provided
5.1 A method for treating multiple sclerosis, e.g. RRMS, or a method for treating an autoimmune disease in a subject in need thereof in a subject in need thereof, comprising administering to the subject, a daily dosage of FTY720 or a pharmaceutically acceptable salt thereof, e.g. of about 0,5mg.
5.2. The method as defined in 5.1, wherein the disease is RRMS.
6.1 A method for assessing the need or suitability of a patient for a treatment regimen as described above (e.g. in any of the specified aspects or embodiments of the invention), comprising the steps of:
   (i) determining whether the patient to be treated with an S1P receptor modulator or agonist of the invention is in a category for which the use of a treatment regimen as described above may be beneficial; and
   (ii) if the patient falls within this category, treating the patient using a treatment regimen as described above.
6.2 The method as defined in 6.1 wherein the patient may be in the above category if he or she suffers from or is susceptible to heart failure, arrhythmias, high grade atrio-ventricular blocks or sick sinus syndrome or has a history of syncopal episodes; or is undergoing beta blocker or anti-arrhythmic treatment, e.g. is under treatment with anti-arrhythmic drugs; or has undergone an interruption or treatment holiday in the maintenance dosage regime e.g. a holiday of greater than 4 days, greater than 6, 8, 10, 12 or 14 days.

The regimen of S1 P receptor modulator or agonist of the invention which is administered to the subject according to the invention may be given either during at the beginning of the disease therapy, e.g. during the initial 10 days, or after an interruption of S1P receptor modulator or agonist therapy, for example an interruption of more than 10 days, more than 12 days, e.g. more than 14 days.

According to the invention the daily dosage of the compound, e.g. FTY720 or FTY720 phosphate, is 0.5mg.

The utility of an S1P receptor modulator or agonist dosage regimen in treating diseases and conditions as hereinabove specified may be demonstrated in standard animal or clinical tests, e.g. in accordance with the methods described hereinafter.

### Brief description of the Figure:

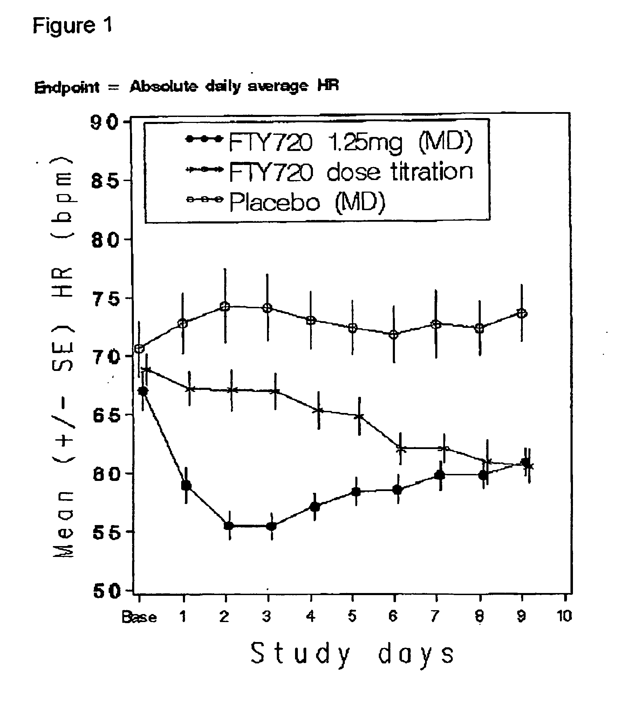

Figure 1 shows the mean (+/- standard error) daily average heart rate on days 1 to 9 for the treatment regimen groups described in the Example below. The x-axis is shows the study days and the Y-axis shows the mean (+/-SE) daily average heart rate (beats per minute (BPM)).

### Example:

A single-center, double-blind, placebo-controlled, dose titration, once-daily, multiple-oral-dose clinical study in healthy subjects was conducted to evaluate the effect of a dosage regimen of FTY720 according to the present invention.

A total of 36 subjects were randomly assigned to one of the three groups.

Each subject participated in a screening period of maximal 21 days, a baseline period (Day -1), a 9-day dose-administration period, and clinical study completion assessments on Day 10. Subjects were assigned to one of the three groups (dose titration regimen group, placebo control group, or active control group) and received the once daily dose in a double-blinded manner (Table 1).

In the dose titration group, increasing once daily doses of Compound A (FTY720), starting at 0.125 mg o.d. and ending at the maximal therapeutic dose of 1.25 mg o.d. were administered on days 1 to 9 of the study according to the schedule shown in Table 1 below. The placebo control group received placebo through out the duration of the study and the active control group received a once daily dose of 1.25 mg of FTY720 on days 1 to 9.

**Table 1**

| **Study group / Number of subjects** | **Baseline** | **Administration period** | | | | | | | | |
|---|---|---|---|---|---|---|---|---|---|---|
| **Day** | **Day -1** | **Day 1** | **Day 2** | **Day 3** | **Day 4** | **Day 5** | **Day 6** | **Day 7** | **Day 8** | **Day 9** |
| **Dose titration regimen group / N = 15** | **Placebo** | **0.125 mg** | **0.125 mg** | **0.125 mg** | **0.25 mg** | **0.25 mg** | **0.5 mg** | **0.5 mg** | **1.25 mg** | **1.25 mg** |
| **Placebo control group / N = 9** | **Placebo** | **Placebo, once daily over 9 days** | | | | | | | | |
| **Active** | **Placebo** | **1.25 mg, once daily over 9 days** | | | | | | | | |
| **Study group / Number of subjects** | **Baseline** | **Administration period** | | | | | | | | |
| **control group / N = 12** | | | | | | | | | | |

On Day -1 (baseline), subjects will undergo baseline assessments including 24 hour holter monitoring and telemetry assessments.

Pharmacodynamic and safety assessments are performed up to 24 hours post last dose. Heart rate is monitored via telemetry on Day -1 through Day 10, 24 hour after the last dosing. Heart rhythm is assessed via 24hr continuous holter monitoring on Day -1, Day 1, and Day 9. For each dosing for each subject, the drug is administered as closely as practically possible to the time administered on Day -1. Safety assessments includes physical examinations, vital signs and body measurements, 12-lead ECG evaluations, standard clinical laboratory evaluations hematology, blood chemistry, urinalysis, adverse event and serious adverse event monitoring. Systolic and diastolic blood pressure and pulse are measured after the subject has rested quietly in the sitting position for at least 3 minutes. Blood pressure is measured at the same arm on each occasion. Standard 12-lead ECGs are recorded at Screening, baseline, 4 hours post-dose on Day 1 and Day 8, and at Study Completion. The variables recorded are: date and time of ECG, heart rate, PR interval, QT interval (uncorrected), QTcB, QRS duration. The overall interpretation is collected with a Yes/No statement to confirm if any clinically relevant abnormalities are present, which needs to be specified further.

Subjects remain on continuous telemetry, starting before breakfast on Day -1 and proceeding throughout the administration period up to Day 10 (24 hour after the last dose). Over this ten day duration of continuous heart rate collection for each subject, one heart rate value is obtained every minute ('minute unit heart rate'), representing the average heart rate value over that minute. The heart rate database for each subject contains approximately 14,400 data points (10 days x 24 hr x 60 min).

24-hour continuous Holter-ECG data are captured via a digital Holter monitor (12-lead, on Days -1, 1, 6, and 8), and transferred for interpretation and reporting. Holter monitoring starts approximately at 7:00 and the time of dose administration is regarded as the time "0 hours". Holter "cuts" are derived from the dataset at 1 hour intervals starting from Day -1 and continuing for 24 hours or the end of the cleaned Holter monitoring dataset.

Cardiac conduction intervals: arrhythmia monitoring includes the frequency and duration of sinus pauses (>2 sec and > 3 sec) and atrio-ventricular blocks. Frequency and duration of atrial and ventricular ectopy and sinus rhythm are also recorded.

The daily chronotropic effect is defined as the percent decrease in HRₘᵢₙ (minimum heart rate) between two consecutive days. It is calculated on days 1 to 9.

### Results:

The results are shown in Figure 1. In the placebo group, daily average heart rate varies by approximately 5 BPM (beats per min) over the course of the study with a trend for heart rate to increase approximately 3-4 BPM from Day -1 to Day 2. The FTY720 1.25 mg treatment group manifestes a significant decrease in heart rate of approximately 8 BPM from Day -1 to Day 1 and an additional decrease in heart rate approximately 3 BPM from Day 1 to Day 2. The FTY720 titration group manifestes a gradual decrease in heart rate of approximately 1-2 BPM per day over the course of eight day course of the dose titration. The initiation of the 1.25 mg FTY720 on Day 8 does not result in dip in heart rate compared to the preceding days.

These results indicate that the use of a dose titration regimen according to the invention attenuates the negative chronotropic effect seen on Day 1 of FTY720 treatment initiation. Furthermore multiple analyses have been conducted to compare minimum heart rates between the two FTY720 treatment group. These analysis show that the FTY720 dose titration regimen provides improved daily minimum heart rates during the course of the study.

## Claims

1. The compound named 2-amino-2-[2-(4-octylphenyl) ethyl]propane-1,3-diol in free form or in a pharmaceutically acceptable salt form, or the phosphate thereof of formula IIa, 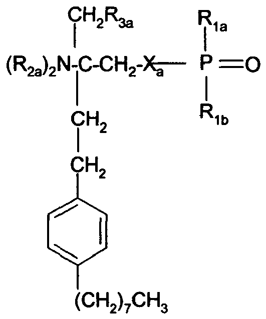 wherein R₂ₐ is H, R₃ₐ is OH, Xₐ is O, R₁ₐ and R_{1b} are OH, for use in the treatment of multiple sclerosis, whereby, during the initial period of treatment said compound is given at a initial daily dosage of 0.125mg and after at a daily dose of 0.25mg, and then the treatment is continued with the standard daily dosage of 0.5mg.

2. The compound according to claim 1 for use in the treatment according to claim 1, wherein the duration of the initial period of treatment is selected from the group consisting of: 4 to 12 days, 5 to 14 days, up to 10 days, 7 to 10 days, 9 days, 8 days, 7 days, 6 days, 5 days or 4 days.

3. The compound according to claim 1 for use in the treatment according to claim 1, wherein the duration of the initial period of treatment is 7 to 10 days.

4. The compound according to claim 1 for use in the treatment according to any one of claims 1 to 3, in a patient at risk of cardiac side effects or heart failure.

5. The compound according to claim 1 for use in the treatment according to any one of claims 1 to 3, while limiting, reducing or preventing the occurrence of symptoms including dizziness, fatigue and heart palpitations.

6. The compound according to claim 1 for use in the treatment according to any one of claims 1 to 5, wherein said compound is administered to patients having undergone an interruption or treatment holiday of therapy with said compound.

7. The compound according to claim 1 for use in the treatment according to any one of claims 1 to 6, wherein multiple sclerosis is relapse remitting multiple sclerosis or primary progressive multiple sclerosis.

8. The compound according to claim 1 for use in the treatment according to any one of claims 1 to 7, wherein the compound is 2-amino-2-[2-(4-octylphenyl) ethyl]propane-1,3-diol in free form or the hydrochloride thereof.

9. A kit comprising units of medication of a compound named 2-amino-2-[2-(4-octylphenyl) ethyl]propane-1,3-diol in free form or in a pharmaceutically acceptable salt form, or the phosphate thereof of formula IIa, 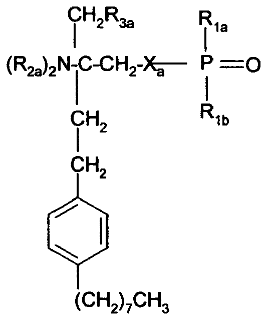 wherein R₂ₐ is H, R₃ₐ is OH, Xₐ is O, R₁ₐ and R_{1b} are OH, for use in the treatment according to claim 1, in which the units of medication contain the daily dosages as defined in claim 1 for administration to the patient during the initial period of treatment.

10. The kit according to claim 9 for use in the treatment according to claim 1, further comprising units for the standard daily dosage of 0.5mg of the compound.

11. The kit according to claim 9 or 10 for use in the treatment according to claim 1, wherein the compound is 2-amino-2-[2-(4-octylphenyl) ethyl]propane-1,3-diol in free form or the hydrochloride thereof.

## Patentansprüche

1. Verbindung mit der Bezeichnung 2-Amino-2-[2-(4-octylphe-nyl)ethyl]propan-1,3-diol in freier Form oder in einer pharmazeutisch annehmbaren Salzform oder das Phosphat davon der Formel IIa 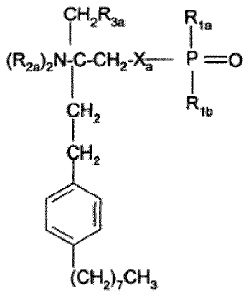 wobei R₂ₐ H ist, R₃ₐ OH ist, Xₐ 0 ist, R₁ₐ und R_{1b} OH sind, zur Verwendung bei der Behandlung von multipler Sklerose, wobei während der Anfangsphase der Behandlung die Verbindung in einer anfänglichen Tagesdosierung von 0,125 mg und danach in einer Tagesdosis von 0,25 mg gegeben wird, und dann die Behandlung mit der Standard-Tagesdosierung von 0,5 mg fortgesetzt wird.

2. Verbindung nach Anspruch 1 zur Verwendung bei der Behandlung nach Anspruch 1, wobei die Dauer der Anfangsphase der Behandlung ausgewählt ist aus der Gruppe bestehend aus: 4 bis 12 Tage, 5 bis 14 Tage, bis zu 10 Tage, 7 bis 10 Tage, 9 Tage, 8 Tage, 7 Tage, 6 Tage, 5 Tage oder 4 Tage.

3. Verbindung nach Anspruch 1 zur Verwendung bei der Behandlung nach Anspruch 1, wobei die Dauer der Anfangsphase der Behandlung von 7 bis 10 Tage beträgt.

4. Verbindung nach Anspruch 1 zur Verwendung bei der Behandlung nach einem der Ansprüche 1 bis 3 bei einem Patienten mit einem Risiko von Herznebenwirkungen oder Herzversagen.

5. Verbindung nach Anspruch 1 zur Verwendung bei der Behandlung nach einem der Ansprüche 1 bis 3, wobei das Auftreten von Symptomen einschließlich Schwindel, Müdigkeit und Herzklopfen begrenzt, verringert oder verhindert wird.

6. Verbindung nach Anspruch 1 zur Verwendung bei der Behandlung nach einem der Ansprüche 1 bis 5, wobei die Verbindung an Patienten verabreicht wird, die eine Therapie mit der Verbindung unterbrochen oder vorübergehend abgesetzt haben.

7. Verbindung nach Anspruch 1 zur Verwendung bei der Behandlung nach einem der Ansprüche 1 bis 6, wobei die multiple Sklerose schubförmig remittierende multiple Sklerose oder primär progrediente multiple Sklerose ist.

8. Verbindung nach Anspruch 1 zur Verwendung bei der Behandlung nach einem der Ansprüche 1 bis 7, wobei die Verbindung 2-Amino-2-[2-(4-octylphenyl)ethyl]propan-1,3-diol in freier Form oder das Hydrochlorid davon ist.

9. Kit, umfassend Einheiten einer Medikation einer Verbindung mit der Bezeichnung 2-Amino-2-[2-(4-octylphenyl)ethyl]propan-1,3-diol in freier Form oder in einer pharmazeutisch annehmbaren Salzform oder das Phosphat davon der Formel IIa 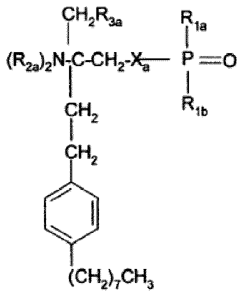 wobei R₂ₐ H ist, R₃ₐ OH ist, Xₐ 0 ist, R₁ₐ und R_{1b} OH sind, zur Verwendung bei der Behandlung nach Anspruch 1, wobei die Einheiten der Medikation die Tagesdosierungen wie in Anspruch 1 definiert zur Verabreichung an den Patienten während der Anfangsphase der Behandlung enthalten.

10. Kit nach Anspruch 9 zur Verwendung bei der Behandlung nach Anspruch 1, weiter umfassend Einheiten für die Standard-Tages-dosierung von 0,5 mg Verbindung.

11. Kit nach Anspruch 9 oder 10 zur Verwendung bei der Behandlung nach Anspruch 1, wobei die Verbindung 2-Amino-2-[2-(4-octylphenyl)ethyl]propan-1,3-diol in freier Form oder das Hydrochlorid davon ist.

## Revendications

1. Composé nommé 2-amino-2-[2-(4-octylphényl)éthyl)-propane-1,3-diol sous forme libre ou sous la forme d'un sel pharmaceutiquement acceptable, ou son phosphate de formule IIa, 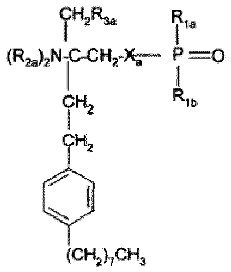 dans laquelle R₂ₐ représente H, R₃ₐ représente un groupe OH, Xₐ représente O, R₁ₐ et R_{1b} représentent un groupe OH, pour une utilisation dans le traitement de la sclérose en plaques, en vertu duquel, durant la période initiale de traitement, ledit composé est administré à une dose quotidienne initiale de 0,125 mg et après à une dose quotidienne de 0,25 mg, et ensuite le traitement est poursuivi avec la dose quotidienne classique de 0,5 mg.

2. Composé selon la revendication 1 pour une utilisation dans le traitement selon la revendication 1, la durée de la période initiale de traitement étant choisie dans le groupe consistant en : 4 à 12 jours, 5 à 14 jours, jusqu'à 10 jours, 7 à 10 jours, 9 jours, 8 jours, 7 jours, 6 jours, 5 jours ou 4 jours.

3. Composé selon la revendication 1 pour une utilisation dans le traitement selon la revendication 1, la durée de la période initiale de traitement étant de 7 à 10 jours.

4. Composé selon la revendication 1 pour une utilisation dans le traitement selon l'une quelconque des revendications 1 à 3, chez un patient à risque de développer des effets secondaires cardiaques ou une insuffisance cardiaque.

5. Composé selon la revendication 1 pour une utilisation dans le traitement selon l'une quelconque des revendications 1 à 3, tout en limitant, en réduisant ou en empêchant l'apparition de symptômes y compris des vertiges, une fatigue et des palpitations cardiaques.

6. Composé selon la revendication 1 pour une utilisation dans le traitement selon l'une quelconque des revendications 1 à 5, ledit composé étant administré aux patients ayant subi une interruption ou une pause thérapeutique de traitement avec ledit composé.

7. Composé selon la revendication 1 pour une utilisation dans le traitement selon l'une quelconque des revendications 1 à 6, la sclérose en plaques étant une sclérose en plaques récurrente rémittente ou une sclérose en plaques primaire progressive.

8. Composé selon la revendication 1 pour une utilisation dans le traitement selon l'une quelconque des revendications 1 à 7, le composé étant le 2-amino-2-[2-(4-octylphényl)éthyl)propane-1,3-diol sous forme libre ou son chlorhydrate.

9. Kit comprenant des unités de médication d'un composé nommé 2-amino-2-[2-(4-octylphényl)éthyl)-propane-1,3-diol sous forme libre ou sous la forme d'un sel pharmaceutiquement acceptable, ou son phosphate de formule IIa, 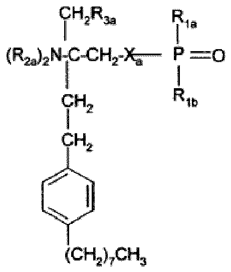 dans laquelle R₂ₐ représente H, R₃ₐ représente un groupe OH, Xₐ représente O, R₁ₐ et R_{1b} représentent un groupe OH, pour une utilisation dans le traitement selon la revendication 1, dans lequel les unités de médication contiennent les doses quotidiennes telles que définies dans la revendication 1 pour une administration au patient durant la période initiale de traitement.

10. Kit selon la revendication 9 pour une utilisation dans le traitement selon la revendication 1, comprenant en outre des unités pour la dose quotidienne classique de 0,5 mg du composé.

11. Kit selon la revendication 9 ou 10 pour une utilisation dans le traitement selon la revendication 1, dans lequel le composé est le 2-amino-2-[2-(4-octylphényl)éthyl)propane-1,3-diol sous forme libre ou son chlorhydrate.
